# EUROPÄISCHE PATENTANMELDUNG

(11) **EP 0 741 295 A2**
(43) Veröffentlichungstag der Anmeldung: **06.11.1996**
(21) Anmeldenummer: 96106305.4
(22) Anmeldetag: 22.04.1996
(51) Int. Cl.: G01N 33/32, B41F 31/00

(54) **Vorrichtung für die Zügigkeitsmessung von pastösen Substanzen**

(30) Priorität: 03.05.1995 DE 19516192
(71) Anmelder: prüfbau Dr.-Ing. H. Dürner GmbH, 82380 Peissenberg (DE)
(72) Erfinder: Wild, Dieter, Prof. Dipl.-Ing., 81475 München (DE)
(74) Vertreter: Zmyj, Erwin, Dipl.-Ing., Dipl.-Wirtsch.-Ing.

(57) **Zusammenfassung**

Die Vorrichtung für die Zügigkeitsmessung umfaßt eine Antriebswalze (11) mit einem Metallmantel (12) sowie eine geringfügig gegenüber dieser bewegbar angeordneten Meßwalze (14), die einen hartelastischen Mantel (15) aufweist, in dessen Oberfläche zumindest ein, vorzugsweise mehrere Temperatursensoren (20, 21) derart eingebettet sind, daß diese Temperatursensoren einen Teil der Walzenoberfläche bilden. Die Temperatursensoren sind durch Leitungen (23) mit einer Auswerteelektronik (19) verbunden, die über mitrotierende Schleifkontakte (17) und einen stationären Schleifkontakte (18) mit einem nicht dargestellten Rechner zur Auswertung der Temperaturwerte verbunden ist. Da die Temperatursensoren in die Oberfläche der Meßwalze eingebettet sind, können sie die Temperatur der zu überprüfenden Substanz, welche auf die Walzen aufgetragen ist, unmittelbar erfassen.

## Beschreibung

Die Erfindung bezieht sich auf eine Vorrichtung für die Zügigkeitsmessung von pastösen Substanzen, insbesondere Druckfarben, mit einer eine Metalloberfläche aufweisenden Antriebswalze und einer mit einem hartelastischem Überzug versehenen Meßwalze, zwischen denen die zu messende Substanz einbringbar und gleichmäßig verteilbar ist, wobei die Meßwalze mit einer Meßeinrichtung für die durch die eingebrachte Substanz verursachten und ein Maß für die Zügigkeit darstellenden Reaktionskräfte ausgerüstet ist.

Bei dem Einsatz von pastösen Substanzen in den verschiedensten Anwendungsfällen ist es erforderlich die rheologischen Eigenschaften zu kennen. Dies gilt insbesondere für den Einsatz von Druckfarben für Offset- und Hochdruck, die in Walzenstuhlanordnungen (z.B. Walzenfarbwerk einer Offsetmaschine) verarbeitet werden. Zur Bestimmung dieser reologischen Eigenschaften dienen sogenannte Zügigkeitsmeßgeräte, z.B. Inkometer, Tack-o-Scope, Inkomat. Diese Meßgeräte sind einem 3-Walzenstuhl nachempfunden, wobei das eigentliche Meßsystem aus einer hartelastisch bezogenen Meßwalze besteht, die im Kraftschluß von einer mit bestimmter Geschwindigkeit umlaufenden Metallwalze angetrieben wird. Eine dritte, entweder angetriebene oder ebenfalls im Kraftschluß mitlaufende Verreibewalze sorgt durch Changierbewegungen für eine gleichmäßige Verteilung der in diese Anordnung für eine Zügigkeitsmessung eingebrachten Substanzmenge.

Im Walzenspalt zwischen Meßwalze und Antriebswalze wird die Substanz Scherkräften unterworfen, im Einlaufbereich des Spaltes komprimiert und im Auslaufbereich gespalten. Die entstehenden Scher-, Druck- und Kavitationsspannungen erzeugen Kraftkomponenten am Umfang der Meßwalze, die in ihrer Summe durch deren Lagerreaktionskraft kompensiert werden. Aus der zu messenden Lagereaktionskraft wird der Zügigkeitswert der zu prüfenden Substanz gewonnen.

Neben den rheologischen Eigenschaften der Substanzen beeinflussen noch folgende Systemgrößen die Zügigkeitsmessung, die im Gegensatz zur gewünschten Meßgröße Störgrößen sind. Es sind dies die geometrischen Abmessungen des Walzensystems, das Gewicht und die Lagerreibung der Meßwalze, die Walzengeschwindigkeit, die Elastizität des Meßwalzenbezuges, die eingebrachte Substanzmenge und die Temperatur der Substanz während der Messung.

Es ist bekannt, den Einfluß der genannten Störgrößen durch Konstanthaltung während der Messung zu minimieren. Gelingt dies, so könnten durch geeignete Kalibrierverfahren die Störgrößen im Rahmen der gewünschten Meßgenauigkeit ausgeschaltet werden. In der Regel gelingt dieses Konstanthalten für die ersten fünf Störgrößen befriedigend. Die Störgröße Substanztemperatur versucht man bisher durch Temperierung der aus Metall bestehenden Antriebswalze konstant zu halten, d.h. man versucht die Systemtemperatur konstant zu halten, was jedoch nicht gelingt, wie sich dies aus den nachfolgenden Darstellungen ergibt.

Aus der Wärmebilanz für das eingangs erläuterte System ergeben sich die nachfolgenden Verhältnisse. Die temperierte Antriebswalze stellt ein Wärmereservoir konstanter Temperatur dar. Die Berührzone zwischen Antriebswalze und der mit hartelastischer Gummimischung bezogenen Meßwalze, die etwa 0,5 bis 1 mm beträgt, wirkt als hoher Wärmeübergangswiderstand zwischen den beiden Walzen, wobei festzuhalten ist, daß der hartelastische Überzug bei der Meßwalze ein schlechter Wärmeleiter ist. Der mit 100 bis 600 m/min Oberflächengeschwindigkeit rotierende freie Umfang der Meßwalze, der etwa 370 mm beträgt, besitzt einen vergleichsweise sehr geringen Wärmewiderstand gegenüber der Umgebungsluft, wodurch ein starker Wärmeaustausch zwischen der Oberfläche der Meßwalze und der Umgebungsluft eintritt. Dabei ist zu berücksichtigen, daß die Temperatur der Umgebungsluft nicht konstant ist. Die tatsächliche Temperatur der Substanzschicht auf dem Walzensystem wird demnach auch bei konstant temperierter Antriebswalze in der Tendenz der Raumtemperatur folgen. Während der aktuellen Messung erfolgt ein weiterer Wärmeeintrag in das System durch Walk- und Scherarbeit in der Berührzone, der in seiner Größe bei sonst gleichen Verhältnissen von der Zähigkeit der zu messenden Substanz abhängt, sowie von der Meßgeschwindigkeit und der Meßdauer. Diese Verlustarbeit führt in jedem Fall zu einer Temperaturerhöhung in der Substanzschicht während der Messung. Die aktuelle Substanztemperatur ergibt sich somit als Folge eines Gleichgewichtszustandes der Wärmeflüsse zwischen Meß- und Antriebswalze sowie zwischen Meßwalze und Umgebungsluft und dem Wärmeeintrag durch die Verlustarbeit in der Berührzone. Hierdurch ergibt sich eine Temperaturerhöhung innerhalb der zu überprüfenden Substanz, so daß es zwangsläufig zu Meßfehlern kommen muß, weil man bisher davon ausgegangen ist, daß die Substanztemperatur während der Messung der Temperatur der temperierten Antriebswalze entspricht. Da aber die rheologischen Eigenschaften solcher pastöser Substanzen sich mit einer Temperaturänderung verändern, ist ersichtlich, daß es zwangsläufig zu Meßfehlern kommen muß.

Aufgabe der Erfindung ist es, die Temperaturänderungen in der zu messenden Substanz während des Meßvorganges zu berücksichtigen um hierdurch zu wesentlich genaueren Zügigkeitsmeßwerten zu gelangen.

Diese Aufgabe wird, ausgehend von einer Vorrichtung der eingangs erläuterten Art erfindungsgemäß dadurch gelöst, daß in die Oberfläche zumindest der Meßwalze zumindest ein Temperatursensor eingebracht ist, der einen Teil der Walzenoberfläche bildet.

Durch diese Ausgestaltung ist eine äußerst genaue Temperaturerfassung der zu überprüfenden Substanz möglich, wodurch die Störgröße, Temperaturveränderung in der Substanz während der Messung, weitgehend ausgeschaltet werden kann. Mit der erfindungsgemäßen Ausgestaltung ist es also möglich die sich während eines Meßvorganges ergebende Temperaturänderung der Meßsubstanz zu erfassen und somit die sich durch Temperaturveränderung ergebenden Änderungen der rheologischen Eigenschaften und damit der Zügigkeit genauer zuzuordnen. Durch die erfindungsgemäße Anordnung zumindest eines Sensors, der Teil der Walzenoberfläche ist, ist gewährleistet, daß dieser Sensor direkt an den Walk- und Spaltvorgängen der eingebrachten Substanz beteiligt ist und somit die genaue Temperatur der Substanz anzeigen kann.

Eine vorteilhafte Weiterbildung der Erfindung besteht darin, daß zumindest ein Temperatursensor in der metallischen Oberfläche der Antriebswalze eingebettet ist und einen Teil der Walzenoberfläche bildet. Hierdurch kann je nach Ausgestaltung, ob der Temperatursensor in wärmeleitendem Kontakt zur Metalloberfläche der Antriebswalze steht oder durch Wärmeisolierung gegenüber der ihn aufnehmenden Walzenoberfläche abgeschirmt ist, entweder die Temperatur der Walzenoberfläche oder die Temperatur der zu überprüfenden Substanz an der der aus Metall bestehenden Antriebswalze zugeordneten Seite der Substanzschicht erfaßt werden. Hierdurch ergibt sich eine weitere Verbesserung der Meßgenauigkeit, da bei der ersten Ausgestaltung genauere Auskünfte in bezug auf die Temperatur des Meßsystems und im zweiten Falle, in welchem der Temperatursensor gegenüber der Metalloberfläche wärmesoliert ist, eine genauere Erfassung der Substanztemperatur ermöglicht wird.

Als Temperatursensoren können Halbleiter, elektronische Widerstände, z.B. NTC- oder PTC- oder PT-Elemente dienen.

Die Erfindung wird nachfolgend von in der Zeichnung dargestellter Ausführungsbeispiele näher erläutert. In der Zeichnung zeigen:
- **Figur 1:**: eine schematische Darstellung einer bekannten Meßvorrichtung im Querschnitt;
- **Figur 2:**: einen Längsschnitt durch eine erste Ausführungsform einer erfindungsgemäßen Vorrichtung; und
- **Figur 3:**: einen Längsschnitt durch eine zweite Ausführungsform einer Meßvorrichtung.

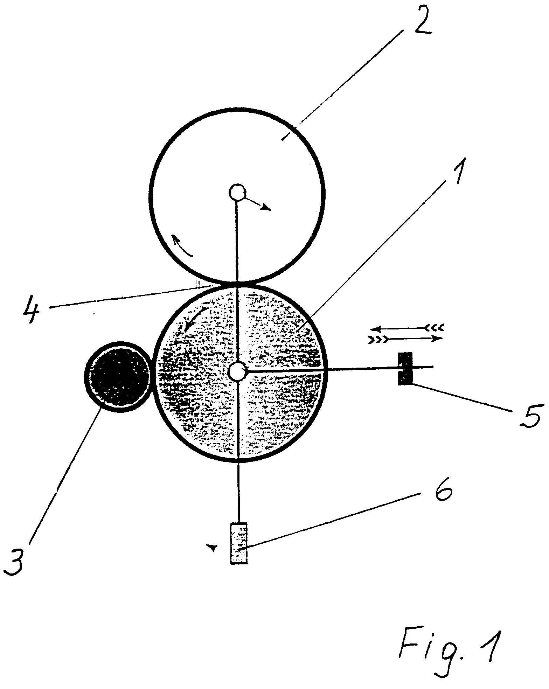

Figur 1 zeigt den schematischen Aufbau einer unter der Bezeichnung "Inkomat" bekannten Meßvorrichtung, bestehend aus einer Antriebswalze 1, einer Meßwalze 2 und einer Verreibewalze 3. Die Antriebswalze 1 weist einen aus Metall bestehenden Mantel auf und ist im Inneren durch eine nicht dargestellte Temperiereinrichtung auf konstanter Temperatur gehalten. Die Meßwalze 2 ist mit einem Mantel aus einer hartelastischen Gummimischung überzogen, so daß hier ein schlechter Wärmeleiter vorliegt. Die Verreibewalze 3 führt Changierbewegungen aus und dient für eine gleichmäßige Verteilung der aufgetragenen Substanz, die im Spalt 4, Scher-, Druck- und Kavitationsspannungen ausgesetzt wird. Die dabei entstehende Lagerreaktionskraft wird durch das Gewicht 5 festgestellt. Mit 6 ist ein Ausgleichsgewicht bezeichnet.

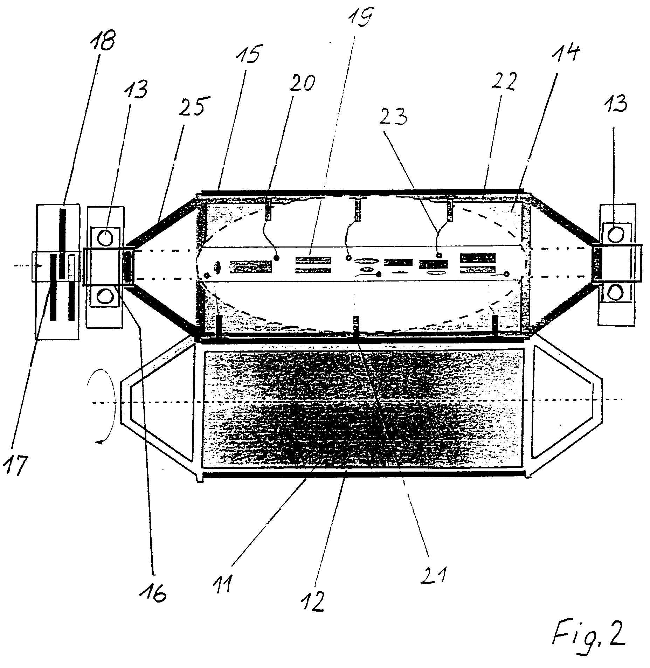

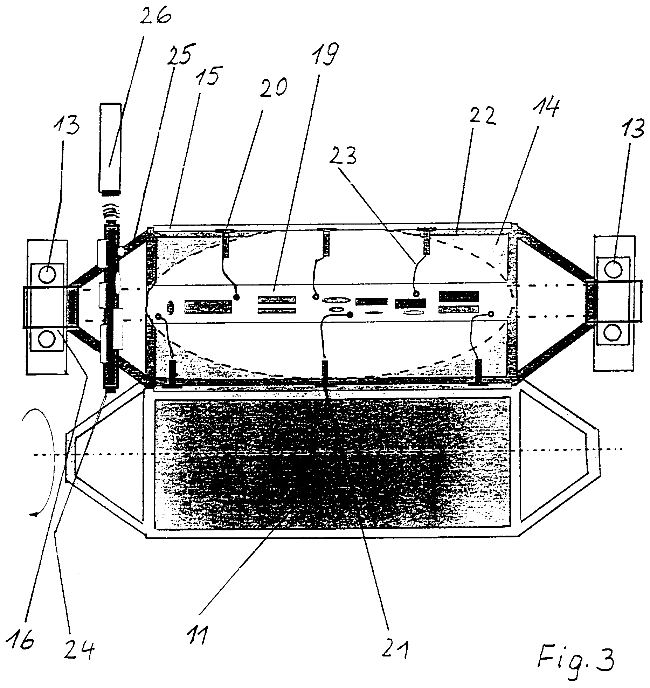

Bei den Vorrichtungen gemäß den Figuren 2 und 3, die bis auf die Meßwertübertragung identisch sind, ist mit 11 eine Antriebswalze bezeichnet, die einen aus Metall bestehenden Mantel 12 aufweist. Über dieser Antriebswalze ist in Lagern 13 eine Meßwalze 14 drehbar und gegenüber der Antriebswalze so gehalten, daß bedingt durch die durch Kraftübertragung über die zwischen den Walzen vorhandene Substanz auftretenden Kräfte die Meßwalze in gewisser Weise gegenüber der Antriebswalze geringfügig im wesentlichen in horizontaler Richtung ausweichen kann. Diese Ausweichbewegung ist ein proportionales Maß für die Zügigkeit der zu messenden Substanz 15. An einem der beiden zur Lagerung der Meßwalze 14 dienenden Enden der Welle 16 sind Schleifringkontakte 17 angeordnet, die mit der Welle 16 mitrotieren und mit einem fest angeordneten Schleifringkontakt 18 zusammenwirken. Diese Schleifringkontakte sind vorgesehen, um Meßwerte einer Auswerteelektronik 19 zu übertragen, die innerhalb der Meßwalze 14 angeordnet ist. Diese Auswerteelektronik überträgt die Temperaturwerte, die von Temperatursensoren 20 und 21 geliefert werden, welche so in der Oberfläche des Mantels 22 der Meßwalze 14 eingesetzt sind, daß sie einen Teil der Oberfläche dieses Mantels 22 bilden und somit direkt in Kontakt mit der zu überprüfenden Substanz 15 stehen. Mit 23 sind Leitungen bezeichnet, die die Temperatursensoren 20 und 21 mit der Auswerteelektronik 19 verbinden. Die von den Temperatursensoren abgegebenen Signale werden von der Auswerteelektronik so geformt, daß keine Störungen durch die Rotation der Meßwalze bei der Weitergabe der Informationen über die Schleifringkontakte einfließen. Diese Auswerteelektronik ist über die Schleifringkontakte mit einem nicht dargestellten Rechner verbunden. Bei der dargestellten Ausführungsform sind die Thermosensoren 20 und 21 an verschiedenen Stellen der Meßwalze angeordnet. Solche Sensoren können auch im Mantel 12 der Antriebswalze angeordnet sein, wobei sie gegenüber diesem Mantel in wärmeleitendem Kontakt stehen oder isoliert sein können. Für den Fall, daß die Temperatursensoren mit dem Mantel 12 der Antriebswalze in wärmeleitendem Kontakt stehen, wird durch diese Temperatursensoren die Temperatur der Walzenoberfläche gemessen. Sind dagegen die Temperatursensoren gegenüber der Manteloberfläche in wärmeleitender Hinsicht isoliert, so wird zusätzlich zu den Temperatursensoren 20 und 21 der Meßwalze die Temperatur der zu überprüfenden Substanz gemesssen. Weitere Temperatursensoren können auch in einer nicht dargestellten Verreibewalze vorgesehen sein, deren grundsätzliche Anordnung sich aus Figur 1 ergibt.

In Figur 3 ist eine Meßvorrichtung dargestellt, die bis auf die Datenübertragung mit derjenigen nach Figur 2 übereinstimmt. Bei dieser Ausführungsform ist an der Meßwalze 14 außerhalb des zylindrischen Bereiches, in welchem der Auftrag der zu überprüfenden Substanz vorgenommen wird, ein ringförmig angeordneter, mitdrehender Infrarot-Sender/Empfänger 24 vorgesehen, der einen kegelstumpfförmigen Übergangsabschnitt 25 umgibt. Der Übergangsabschnitt 25 trägt den Wellenstumpf 16 der im Lager 13 gehalten ist. Ein statisch angeordneter Empfänger/Sender 26 bildet die Kopplung zu einem nicht dargestellten Rechner.

Die in den Figuren 2 und 3 dargestellten Meßwalzen sind genau gewuchtet und die elektronischen Bauteile sind so angeordnet, daß Fehler durch Unwucht ausbleiben. Beim Ablauf der Messung erfolgt gleichzeitig eine Zählung der Drehung bzw. eine genaue Aufzeichnung der Position der Meß- und Antriebswalze. Jeder Meßwert der Temperatursensoren wird zugleich mit einer Positionszahl versehen, wodurch bei Berührung eines Temperatursensors durch die Substanz innerhalb der Kontaktzone zwischen der Meßwalze und der Antriebswalze nicht nur der Temperaturwert registriert, sondern auch einer bestimmten Position zugeordnet wird. Es ergibt sich somit eine genaues Temperaturbild der aufgetragenen Substanz, deren Schichtdicke im Bereich der Berührungszone, die quer zur Längsachse etwa 0,5 bis 1 mm beträgt, im Bereich von wenigen µm liegt, z.B. zwischen 5-20µm, welche gleichzeitig, je nach Spaltungsfaktor, die Summe der jeweiligen Substratschichten auf der Antriebs- bzw. Meßwalze bildet. Die Daten werden mittels eines Rechners ausgewertet.

Neben der Temperatur der temperierten Antriebswalze nicht aber der Walzenoberfläche, d.h. der Temperierflüssigkeit, die bisher schon bei den bekannten Geräten festgestellt und nach Möglichkeit konstant gehalten wurde, erhält man durch die erfindungsgemäße Anordnung ein Informationsbild bestehend aus den Geräteparametern, der Position der Temperatursensoren, der Temperatur in der zu messenden Schicht an der Meßwalze, bezogen auf die einzelnen Temperatursensoren und die Temperaturen der Antriebswalze und der Meßwalze. Experimentell hat sich gezeigt, daß eine Temperierung der Antriebswalze, wie bisher üblich, unter keinen Umständen eine ausreichende Konstanz, bzw. Reproduzierbarkeit der Substanztemperatur während der Zügigkeitsmessung gewährleistet. Durch die Feststellung der tatsächlich bestehenden Temperaturverhältnisse in der Substanz kann auch die Temperatur der Antriebswalze insbesondere die Oberflächentemperatur nachgeregelt werden, woraus sich eine zusätzliche Reduzierung der temperaturbedingten Störeinflüsse bei der Zügigkeitsmessung erreichen läßt.

Der Mitnahmeeffekt bzw. die Auslenkung der beweglich gelagerten und reitenden Meßwalze ergibt abhängig von der grundsätzlichen Zügigkeit der Substanz und der Temperatur der Substanz den Zügigkeitsmeßwert. Über einen vorgegebenen Algorithmus kann somit unter Berücksichtigung der Geräteparameter, der unterschiedlichen Walzengeometrien und variablen Geschwindigkeiten, welche als momentane Festwerte dem Rechner ebenfalls zur Verfügung stehen, ein extrem genaues, von temperaturbedingten Meßfehlern befreites, Meßergebnis erzeugt werden.

Durch die erfindungsgemäße Ausgestaltung kann der bisher bis zu 15% betragende temperaturbedingte Meßfehler auf einen Wert von ca. ± 0,5% gesenkt werden, wodurch sich eine Verbesserung der Zügigkeitsmessung um einen Faktor von ca. 5 erreichen läßt. Dadurch läßt sich die Qualität der Substanz erhöhen und die Qualitätssicherung wird im Sinne von ISO 9000 auf ein sicheres Fundament gestellt. Diese Verbesserung ist vor allem im Laborbereich anzutreffen, da sich zu den prinzipiellen Temperaturveränderungen durch den Arbeitsablauf während des Meßvorganges noch Einflüsse und enorme Fehler ergeben, z.B. durch eine geöffnete Labortür des Klimaraumes, durch Sonneneinstrahlung auf die Meßwalzen usw.

Jeder pastösen Substanz, gleichgültig, ob es sich um eine Farbe oder um eine sonstige Substanz handelt, läßt sich durch die erfindungsgemäße Ausgestaltung der Meßvorrichtung, insbesondere in Abhängigkeit von der Temperatur der zu messenden Substanz eine exakte Zügigkeitsklasse zuordnen. Es läßt sich somit ein exakter Temperaturkoeffizient für die Zügigkeit einer Substanz bestimmen.

Bei der vorliegenden Erfindung zur Temperaturkorrektur der Zügigkeitsmeßwerte wird nun nach folgendem Ansatz verfahren. Bei den temperaturbedingten Fehlmessungen der Zügigkeitswerte handelt es sich um systematische Fehler. Nach den Regeln der Fehlerrechnung sind aber Meßgrößen, die mit systematischen Fehlern behaftet sind, vollkommen korrigierbar, sofern es eindeutige Zusammenhänge zwischen den fehlererzeugenden Störgrößen und der Meßgröße gibt.

Diese Zusammenhänge werden durch die Erfassung der tatsächlichen Temperaturverhältnisse offengelegt.

Einflüsse auf die Zügigkeit einer Substanz ergeben sich weiterhin unter anderem durch die Schichtdicke der auf die Walzen aufgetragenen Substanz. Sie muß auf den Walzen konstant gehalten werden. Der Auftrag auf dem Walzensystem erfolgt zur Zeit mittels einer Pipette. Dies erfordert sorgfältiges Arbeiten. Rüstet man die erfindungsgemäße Meßvorrichtung mit einer integrierten elektronischen Feinstwaage zur Wägung der Pipette aus, so können ungleiche Befüllungen, die sich in unterschiedlichem Gewicht äußern, ausgeschieden werden. Da Gewichtsschwankungen ebenfalls einen systematischen, wenn auch kleinen Fehler darstellen, kann durch die Verwendung einer elektronischen Feinstwaage die Meßgröße der Zügigkeit verbessert werden. Hierzu ist der Rechner direkt mit der Waage verbunden. Die so ausgerüstete Meßvorrichtung ist also in der Lage, unter den gegebenen Bedingungen eine exakte substanzspezifische Kennlinie der Zügigkeit zu errechnen und auszugeben. Alle Informationen lassen sich datentechnisch speichern, voreinstellen und beliebig verarbeiten. Vergleiche unter den verschiedenen Substanzen sind somit in einfacher Weise möglich.

## Patentansprüche

1. Vorrichtung für die Zügigkeitsmessung von pastösen Substanzen, insbesondere Druckfarben, mit einer eine Metalloberfläche aufweisenden Antriebswalze und einer mit einem hartelastischen Überzug versehenen Meßwalze, zwischen denen die zu messende Substanz einbringbar und gleichmäßig verteilbar ist, wobei die Meßwalze mit einer Meßeinrichtung für die durch die eingebrachte Substanz verursachten und ein Maß für die Zügigkeit darstellenden Reaktionskräfte ausgerüstet ist, **dadurch gekennzeichnet**, daß in die Oberfläche zumindest der Meßwalze zumindest ein Temperatursensor (20) eingebracht ist, der einen Teil der Walzenoberfläche bildet.

2. Vorrichtung nach Anspruch 1, **dadurch gekennzeichnet**, daß zumindest ein Temperatursensor in der metallischen Oberfläche (12) der Antriebswalze (11) eingebettet ist und einen Teil der Walzenoberfläche bildet.

3. Vorrichtung nach Anspruch 2, **dadurch gekennzeichnet**, daß der Temperatursensor in wärmeleitenden Kontakt zur Metalloberfläche (12) der Antriebswalze (11) steht.

4. Vorrichtung nach Anspruch 2, **dadurch gekennzeichnet**, daß der Temperatursensor durch Wärmeisolierung gegenüber der ihn aufnehmenden Walzenoberfläche abgeschirmt ist.

5. Vorrichtung nach einem der Ansprüche 1 bis 4, **dadurch gekennzeichnet**, daß als Temperatursensoren temparturempfindliche Halbleiter, elektronische Widerstandselemente, z.B. NTC- oder PTC- oder PT-Elemente dienen.
